# EUROPÄISCHE PATENTSCHRIFT

(11) **EP 0 838 610 B1**
(45) Veröffentlichungstag und Bekanntmachung des Hinweises auf die Patenterteilung: **25.07.2001**
(21) Anmeldenummer: 97121512.4
(22) Anmeldetag: 14.10.1995
(51) Int. Cl.: F16H 31/00

(54) **Antrieb für einen rotierenden Gegenstand wie Walze, Welle oder dergleichen**
Drive for rotating device like a roll, shaft or similar
Entraînement d'un élément rotatif comme un rouleau, un arbre ou similaire

(30) Priorität: 17.10.1994 DE 4437019; 05.11.1994 DE 4439555; 18.11.1994 DE 4441105
(43) Veröffentlichungstag der Anmeldung: 29.04.1998
(62) Teilanmeldung aus: 95936493.6
(73) Patentinhaber: VENTA VERTRIEBS AG, 6331 Hünenberg (CH)
(72) Erfinder: Hitzler, Alfred, D-88284 Mochenwangen (DE); Harter, Erich, D-88284 Mochenwangen (DE)
(74) Vertreter: Patentanwälte Eisele, Otten, Roth & Dobler

(56) Entgegenhaltungen:
- DE-A- 2 424 140
- DE-C- 3 412 745
- FR-A- 1 191 751
- FR-A- 2 595 839
- US-A- 3 465 605
- US-A- 4 261 930
- US-A- 4 536 626

## Beschreibung

Die Erfindung betrifft einen Antrieb für einen rotierenden Gegenstand nach dem Oberbegriff des Anspruchs 1.

Beim Antrieb von Walzen, Wellen oder dergleichen, insbesondere auch beim Antrieb eines Plattenstapels eines Luftbefeuchtungs- und Reinigungsgerätes, eines Duftverdunsters oder dergleichen bei welchem der Plattenstapel innerhalb einer Flüssigkeit rotiert, treten immer wieder Probleme wegen Verunreinigungen des Getriebes durch die entsprechende Flüssigkeit auf.

Dies ist insbesondere dann von Nachteil, wenn es sich um aggressive Flüssigkeiten handelt, z. B. bei einem Duftverdunster liegen derartige Flüssigkeiten beispielsweise in Form von ätherischen Ölen vor. Generell stellt sich dieses Problem jedoch beispielsweise auch im Bereich der Chemie, wo häufig mit aggressiven Flüssigkeiten, insbesondere Säuren und Laugen, hantiert wird.

Umfangreiche Abdichtungsmaßnahmen der Getriebeeinheit führen nicht immer zum gewünschten Erfolg. Häufig bleibt als einzige Lösungsmöglichkeit, sämtliche Getriebekomponenten aus Materialien herzustellen, die gegenüber den verwendeten aggressiven Flüssigkeiten resistent sind.

Dies zieht in der Regel einen erhöhten Kostenaufwand nach sich, wobei immer noch das Problem verbleibt, daß die Getriebeeinheit durch die jeweils verwendete Flüssigkeit verschmutzt wird.

Insbesondere beim Wechsel der Flüssigkeit, d. h. z. B. bei einem Duftverdunster bei einem Wechsel des Duftstoffbades, kann es zu unerwünschten Vermischungen von Flüssigkeiten kommen. Nicht zuletzt bei chemischen Produktionsanlagen kann es hierbei sogar zu unerwünschten chemischen Reaktionen kommen, sofern das Getriebe nicht äußerst sorgfältig gereinigt wird.

Aus der Druckschrift FR-A-1 191 751 ist ein Antrieb nach dem Oberbegriff des Patentanspruchs 1 bekannt geworden. Nachteilig an diesem Antrieb für einen rotierenden Gegenstand ist die Tatsache, dass die in Eingriff stehenden Zähne eines Hebelantriebs mit den zugehörigen Zähnen des anzutreibenden Zahnrades in einer stets kraftschlüssigen und formschlüssigen Verbindung stehen, wobei ein unruhiger und abgehakter Vorschub mit einem hohen Verschleiß der Zahnanordnung insbesondere der Hebel bewirkt wird. Insbesondere ist keine selbständige Führungsanordnung der Hebel vorgesehen, die unabhängig vom Eingriff der jeweiligen Zähne erfolgt.

Eine ähnliche Anordnung ist aus den Dokumenten DE-A-24 24 140 sowie US-A-4 536 626 vorgesehen. Dabei betrifft die erste Druckschrift keinen Antrieb, sondern eine Exzenter gesteuerte Schaltklinke für ein Schrittschaltwerk, das heißt der Bewegungsablauf eines angetriebenen Zahnrads wird durch eine Schaltklinke beeinflußt. Das Dokument US-A-4 536 626 weist ebenfalls einen Antriebsmechanismus mit einem Hebelsystem auf, wobei der Hebel wenigstens einseitig an einer Führungsfläche geführt ist. Die in Eingriff stehenden Zähne werden jedoch wiederum mittels einer Federanordnung zu einem ständigen Eingriff zueinander gezwungen, was zu einem unbefriedigenden Ablauf der Bewegung führt.

Der Erfindung liegt die Aufgabe zugrunde, einen unempfindlichen Antrieb zu schaffen, der auch eine problemlose Trennung von Antrieb und angetriebenen Mittel, z. B. Plattenstapel, erlaubt. Diese Aufgabe wird ausgehend von einem Antrieb nach dem Oberbegriff des Anspruchs 1 durch die kennzeichnenden Merkmale des Anspruchs 1 gelöst.

In den Unteransprüchen sind vorteilhafte und zweckmäßige Weiterbildungen des Gegenstandes nach Anspruch 1 angegeben. Bei einem anzutreibenden Gegenstand, wie z. B. einem Duftverdunster empfiehlt es sich daher, ebenso wie allgemein in jedem Anwendungsgebiet, in dem ein Gegenstand, wie eine Walze, Welle oder dergleichen, bei einem Duftverdunster beispielsweise der Plattenstapel, in einer aggressiven Flüssigkeit rotiert, einen Antrieb vorzusehen, der wenigstens einen Hebel und ein Zahnrad umfaßt, wobei das Zahnrad drehfest mit dem rotierenden Gegenstand verbunden ist. Außerdem wird eine Vorrichtung zur Lagerung und Führung des Hebels für eine in das Zahnrad eingreifende und mitnehmende Vorwärtsbewegung des Hebels vorgesehen.

Selbst wenn das Zahnrad hierbei durch die Rotationsbewegung in der Flüssigkeit von dieser benetzt wird, besteht der einzige Berührungspunkt mit den weiteren Getriebekomponenten in dem Teil des Hebels, der in das Zahnrad eingreift. Dieser Teil des Hebels ist in der Regel jedoch einfach zu reinigen. Außerdem kann die Bewegung des Hebels so geführt und gesteuert werden, daß für den Fall, daß er tatsächlich mit der Flüssigkeit in Berührung kommt, diese Flüssigkeitsreste in Richtung der Hebelspitze ablaufen und somit in der Tat nur ein äußerstes Ende des Hebels von der Flüssigkeit benetzt wird.

Hierzu wird vorzugsweise der Hebel so orientiert und ausgebildet, daß er mit seiner zum Zahnrad hinweisende Spitze in das Zahnrad eingreift und diese Spitze in jeder Stellung des Hebels nach unten ausgerichtet ist.

Vorteilhafterweise wird die Vorrichtung zur Lagerung und Führung des Hebels so eingerichtet, daß sie eine zwischen zwei Vorwärtsbewegungen des Hebels stattfindende Rückwärtsbewegung erlaubt, bei der sich der Hebel nicht mehr in Eingriff mit dem Zahnrad befindet und dieses nicht berührt.

Somit kann der Hebel das Zahnrad kontinuierlich in einer Drehrichtung antreiben. Zudem kann hierdurch unter anderem auf einen Klinkenantrieb verzichtet werden, der nach Art einer Ratsche in der Rückwärtsbewegung mit seiner in der Vorwärtsbewegung in das Zahnrad eingreifenden Klinke über die Zähne des Zahnrades rutscht. Zum einen entfällt dabei ein für ein Klinkenantrieb zusätzlich erforderliches bewegliches Bauteil, zum anderen wird die in Wohnräumen doch sehr störende Geräuschentwicklung eines Klinkenantriebs vollständig vermieden.

Vorzugsweise umfaßt die Vorrichtung zur Lagerung und Führung des Hebels Gleitflächen, die am Hebel selbst angebracht sind und eine Halterung mit Führungsflächen. Hierdurch kann der Hebel außer einer Schwenkbewegung auch eine Translationsbewegung durchführen, die der Schwenkbewegung überlagert ist. Durch die Überlagerung einer Dreh- und einer Translationsbewegung ist es möglich, die Spitze so anzusteuern, daß sie in Vorwärtsbewegung in das Zahnrad eingreift und in der Rückwärtsbewegung außer Eingriff mit diesem steht.

Vorteilhafterweise wird der Hebel an einem Ende mit einer rotierenden Antriebswelle exzentrisch gelenkig verbunden. Durch eine derartige exzentrisch gelenkige Verbindung mit einer rotierenden Welle wird der Hebel gleichzeitig in eine Schwenk- und Translationsbewegung versetzt, die einander überlagert sind. In Verbindung mit den genannten Gleit- und Führungsflächen kann hierbei nahezu jede beliebige zyklische Bewegung, beispielsweise eine Ellipsenbewegung, der Hebelspitze bewirkt werden. Hierzu müssen die Formen der Gleit- bzw. Führungsflächen sowie die Exzentrizität des Hebelantriebs aufeinander abgestimmt werden.

Vorzugsweise werden bei einem erfindungsgemäßen Antrieb zwei Hebel vorgesehen, die abwechselnd in das Zahnrad eingreifen. Dadurch befindet sich ständig ein Hebel in Eingriff mit dem Zahnrad, so daß dieses nicht nur angetrieben, sonderen auch gegen jede unerwünschte weitere Bewegung, beispielsweise gegen ein Zurückdrehen des rotierenden Gegenstandes nach dem Herausschwenken einer Hebelspitze aus dem Zahnrad, verriegelt ist. Die Rotation des rotierenden Gegenstandes ist somit ständig mechanisch fest durch den Hebelantrieb kontrolliert und gesteuert.

Vorzugsweise werden die beiden Hebel unter 180° versetzt an derselben Antriebswelle exzentrisch angelenkt. Wenn beide Hebel gleichgeformte Gleitflächen aufweisen und in entsprechenden Führungsflächen gleiten, vollführen sie dementsprechend identische Vorwärt- und Rückwärtsbewegungen, die um eine halbe Zykluslänge zeitlich zueinander versetzt durchgeführt werden.

In einer bevorzugten Ausführungsform wird die Antriebswelle für den exzentrischen Antrieb des Hebels mit einem Schneckenrad ausgestattet, in das eine Schnecke eingreift. Hierdurch sind zum einen extreme Übersetzungen möglich, was insbesondere bei der Verwendung in einem erfindungsgemäßen Duftverdunster, wo ein Motor für den Ventilator und die Rotation des Plattenstapels eingesetzt wird, notwendig ist. Zum anderen stellt ein Schneckenradantrieb ein sogenanntes selbsthemmendes Getriebe dar, wodurch in Verbindung mit dem Zwei-Hebelsystem, bei dem sich ständig ein Hebel mit dem Zahnrad des rotierenden Gegenstandes in Eingriff befindet, eine Verriegelung des rotierenden Gegenstandes, beispielsweise eines Plattenstapels, bei ausgeschaltetem Antrieb ergibt.

Vorzugsweise wird die Schnecke über eine elastische Kupplung der Antriebswelle eines Motors verbunden. Hierdurch braucht die Antriebswelle nicht vollständig exakt in Flucht mit der Welle der Schnecke zu stehen. Größere Toleranzen bei der Fertigung eines erfindungsgemäßen Antriebs sind hierbei möglich, was unter anderem eine Kostenersparnis mit sich bringt.

Ein Ausführungsbeispiel der Erfindung ist in der Zeichnung dargestellt und wird anhand der nachfolgenden Beschreibung näher erläutert.

Im einzelnen zeigen
- Fig. 1: einen erfindungsgemäßen Antrieb am Beispiel eines Duftverdunsters;
- Figuren 2 und 3: einen schematischen Querschnitt durch ein erfindungsgemäßes Antriebssystem in verschiedenen Hebelpositionen;
- Fig. 4: einen schematischen vertikalen Querschnitt durch einen Teil der Antriebsvorrichtung und
- Fig. 5: eine schematische Darstellung eines Duftverdunsters mit zwei Plattenstapel und doppeltem Antrieb.

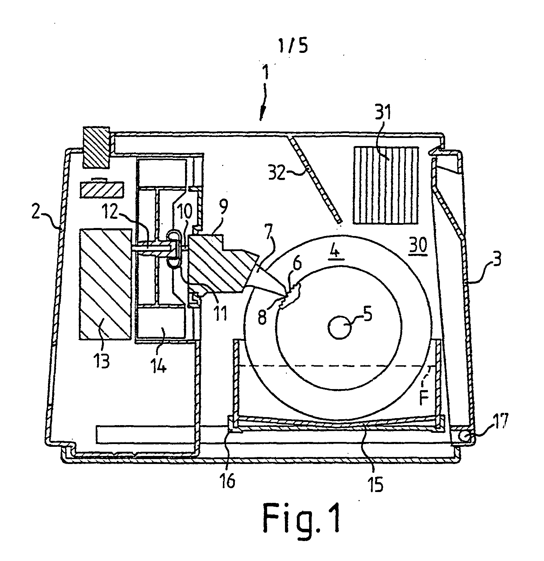

Duftverdunster 1 gemäß Fig. 1 weist ein Gehäuse 2 mit Öffnungsklappe 3 auf. Im Inneren des Gehäuses 2 ist ein Plattenstapel 4 aus lamellenförmig hintereinander angeordneten kreisförmigen Scheiben, dargestellt. In der gezeigten Darstellung ist hierbei nur die vorderste Platte sichtbar. Mit der Achse 5 des Plattenstapels 4 ist eine Zahnscheibe oder Zahnrad 6 fest verbunden. In das Zahnrad 6 greift ein Hebel 7 mit seiner Hebelspitze 8 ein. Das hintere Ende des Hebels 7 ist in einem Getriebegehäuse 9 verborgen. Das Getriebegehäuse 9 wird von einer Getriebeantriebswelle 10 durchsetzt, die über eine elastische Kupplung 11 mit einer Motorantriebswelle 12 eines Elektromotors 13 verbunden ist. Auf der Motorantriebswelle 12 ist ein Flügelrad 14 als Ventilator aufgesetzt.

Der Plattenstapel 4 ist mit seiner Achse 5 in den nicht dargestellten Stirnseiten einer Duftstoffwanne 15 drehbar gelagert. Die Duftstoffwanne 15 steht in einer verschiebbaren Schubladenhalterung 16, an der, wie nicht näher dargestellt, auch die Öffnungsklappe 3 an ihrer Drehachse 17 schwenkbar befestigt ist.

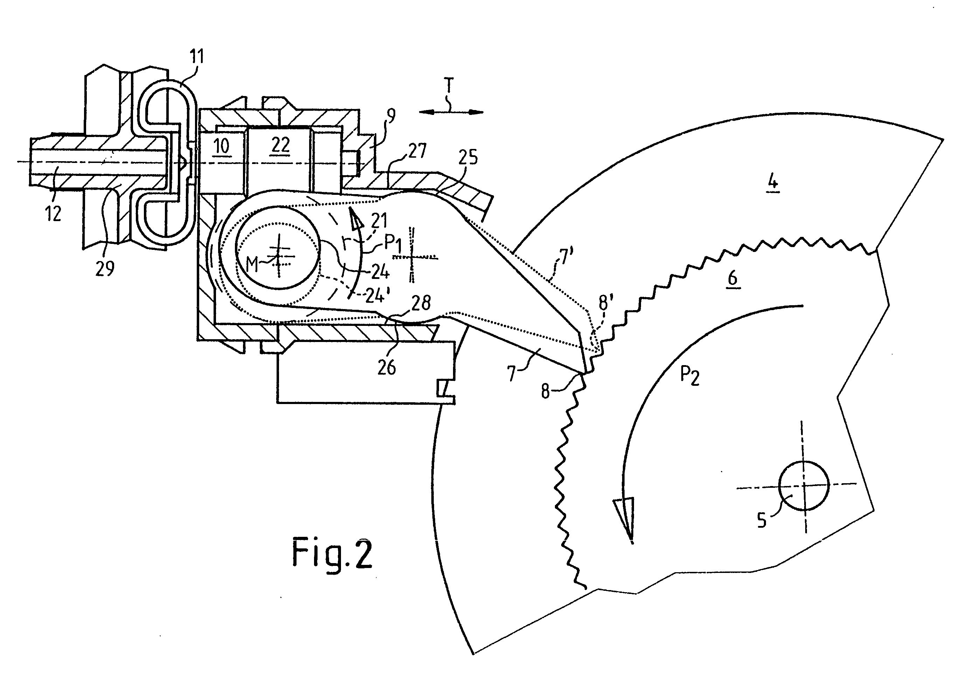

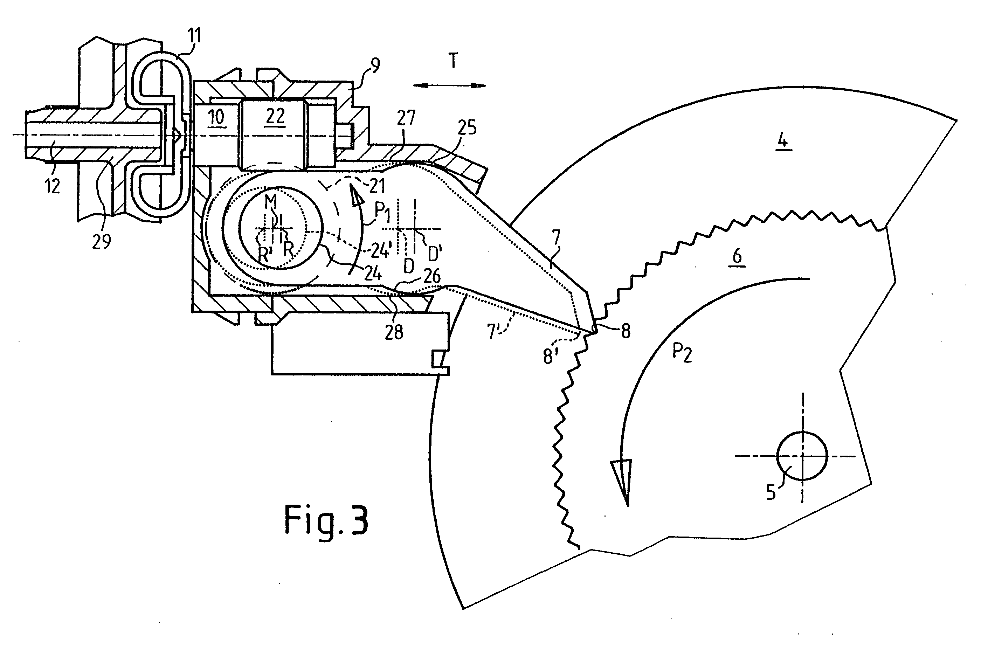

Die Figuren 2 und 3 zeigen einen erfindungsgemäßen Hebelantrieb in vergrößerter Darstellung. Ein vorderer Hebel 7 ist hierbei mit durchgezogenen Linien dargestellt, während ein zweiter hinter dem Hebel 7 befindlicher Hebel 7' mit punktierten Linien gekennzeichnet ist.

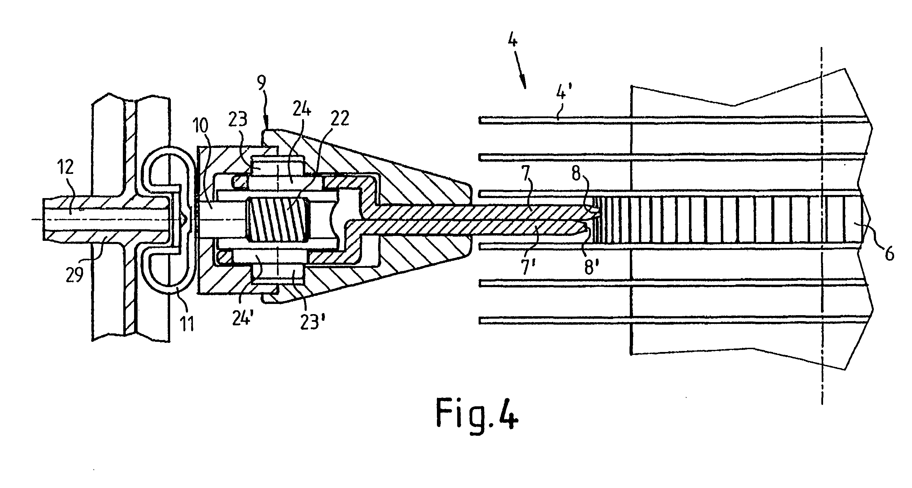

Ein Schneckenrad 21, das eine Schnecke 22 kämmt, ist mit gestrichelten Linien gezeichnet. Das Schneckenrad 21 ist drehfest mit einer in den Figuren 2 und 3 nicht näher dargestellten, jedoch in Fig. 4 erkennbaren Exzenterantriebswelle 23 drehfest verbunden oder in diese eingearbeitet. Auf der Exzenterantriebswelle 23 sitzen zwei als Ringschultern ausgebildete Exzenter 24, 24', deren Mittelachsen R, R' gegenüber der Mittelachse M der Exzenterantriebswelle 23 versetzt sind, so daß sich eine Exzentrizität ergibt. Die Exzenter 24, 24' durchsetzen zwei entsprechende Bohrungen in dem jeweiligen Hebel 7, 7'. Jeder Hebel 7 bzw. 7' ist jeweils mit einer oberen 25 und unteren 26 Gleitfläche versehen. Diese liegen auf oberen 27 und unteren 28 Führungsflächen auf, die in das Getriebegehäuse 9 eingearbeitet sind.

Fig. 4 zeigt die drehfest mit der Getriebeantriebswelle 10 verbundene oder in diese eingearbeitete Schnecke 22. Die elastische Kupplung 11 ist über eine Steckhülse 29 des Flügelrads 14 mit der Motorantriebswelle 12 verbunden. Außerdem ist hier die lamellenartige Anordnung der einzelnen Scheiben 4' des Plattenstapels 4 ersichtlich.

Im Betrieb des Duftverdunsters 1 befindet sich in der Duftstoffwanne 15, wie mit der Linie F als Flüssigkeitspegel dargestellt ist, ein flüssiger Duftstoff. Innerhalb des Duftstoffes liegt teilweise ein Plattenstapel 4. Dieser ist über seine Achse 5 drehbar in der Duftstoffwanne 15 gelagert. Er wird über die Hebel 7, 7' und das Zahnrad 6 in eine Drehbewegung versetzt. Hierbei kommt die mit Duftstoff benetzte Oberfläche des Plattenstapels 4 mit der Luft im Verdunstungsraum 30 in Berührung, so daß der Duftstoff von der Oberfläche in die Luft des Verdunstungsraums 30 verdunstet.

Durch das Flügelrad 14 wird über eine nicht näher dargestellte Öffnung im Gehäuse 2 des Duftverdunsters 1 Luft angesaugt und über den Plattenstapel 4 geblasen. Hierdurch kommt es zu einer verstärkten Verdunstung von Duftstoff. Die mit Duftstoff angereicherte Luft im Verdunstungsraum 30 entweicht anschließend durch entsprechende Lüftungsöffnungen, wie sie durch die Lüftungsschlitze 31 schematisch angedeutet sind. Eine Blende 32 lenkt hierbei den Luftstrom zusätzlich in Richtung auf den Plattenstapel ab.

Falls der Duftstoff gewechselt werden soll, so kann die Klappe 3, wie in Fig. 2 dargestellt, nach unten heruntergeklappt werden und die Duftstoffwanne 15 auf der Schubladenhalterung 16 herausgezogen werden. Der Plattenstapel 4 bleibt hierbei in der Duftstoffwanne 15 gelagert. Anschließend kann die Wechselkassette 18, die die Duftstoffwanne 15 mitsamt ihres Plattenstapels 4 umfaßt, aus der Schubladenhalterung 16 entnommen werden und mit einer Kassettenhaube 19 verschlossen werden. Diese Wechselkassette 18 ist somit bereit für eine längere Lagerung oder für einen Transport. Eine andere baugleiche Wechselkassette oder auch dieselbe Wechselkassette kann anschließend wieder in den Duftverdunster 1 in umgekehrter Reihenfolge eingesetzt werden.

Der Antrieb für den Plattenstapel 4 geschieht über das Zahnrad 6 und die Hebel 7, 7'. Die Hebel 7, 7' werden mit Hilfe des Elektromotors 13, der auch das Flügelrad 14 treibt, angetrieben. Dies geschieht über die Steckhülse 29 des Flügelrads 14 und die elastische Kupplung 11, wodurch die Getriebeantriebswelle 10 angetrieben wird.

Hierdurch dreht sich die Schnecke 22 und somit auch die Exzenterantriebswelle 23 in Richtung des Pfeils P₁. Die Exzenter 24 vollführen dabei eine kreisförmige Bewegung. Durch diese kreisförmige Bewegung werden die Hebel 7, 7' sowohl in eine Kippbewegung um den Drehpunkt D, D' als auch in eine Translationsbewegung in Richtung des Doppelpfeils T versetzt. Während dieser Bewegungen wird jeder Hebel durch die obere und untere Führungsfläche 26, 28 geführt, auf denen er mit seinen oberen bzw. unteren Gleitflächen 25, 26 aufliegt. Durch die besondere Formgebung der Führungsflächen 27, 28 sowie der Gleitflächen 25, 26 in Verbindung mit der Auslenkung der Mittelachsen R, R' der Exzenter 24, 24' von der Mittelachse M der Drehachse 23 ist nahezu jede beliebige Bahnkurve der Hebelspitzen 8, 8' möglich. Von großem Vorteil ist hierbei eine elliptische Bahnkurve, so daß die Hebelspitzen 8, 8' in der Vorwärtsbewegung, d. h. in der vorliegenden Darstellung in der Bewegung nach unten, sich in Eingriff mit dem Zahnrad 6 befinden. Bei der Rückwärtsbewegung, d. h. vorliegend bei der Bewegung nach oben, befinden sich die Hebelspitzen 8, 8' sodann außer Eingriff mit dem Zahnrad 6 und berühren dessen Zähne nicht.

Die Darstellungen der Figuren 2 + 3 zeigen die Hebel 7, 7' in verschiedenen Stellungen während des Antriebsvorgangs, bei dem der Plattenstapel 4 in Richtung des Pfeils P₂ gedreht wird. Da die Auslenkung der Exzenter 24, 24' gegenüber der Mittelachse M der Exzenterantriebswelle 23 um 180° zueinander versetzt liegt, d. h. die Mittelachsen R, R' der Exzenter 24, 24' und die Mittelachse M der Exzenterantriebswelle 23 liegen auf einer Geraden, vollzieht sich die Bewegung des Hebels 7' um einen halben Bewegungszyklus versetzt zur Bewegung des Hebels 7. Hierdurch befindet sich jeweils mindestens eine Hebelspitze 8 in Eingriff des Zahnrads 6, so daß dieses nicht nur in Richtung P₂ mitgenommen, sondern auch in jeder Stellung des Antriebs gegenüber anderen Bewegungen, beispielsweise einem Zurückdrehen entgegen dem Drehsinn von P₂ verriegelt ist.

Die Hebelspitzen 8, 8' sind nach unten abwärts gerichtet, so daß Flüssigkeit, die durch das Eintauchen des Zahnrads 6 in den Duftstoff innerhalb der Duftstoffwanne 15 eventuell zu den Hebelspitzen 8, 8' gefördert wird, wieder hinunter auf das Zahnrad 6 abläuft. Die einzigen Stellen, die also somit mit Duftstoff in Verbindung kommen können, sind lediglich die Spitzen der Hebel 7, 7'. Es besteht somit keine Gefahr, daß die restlichen Komponenten des Antriebs in irgendeiner Phase des Betriebs des Duftverdunsters mit flüssigem Duftstoff in Berührung kommen.

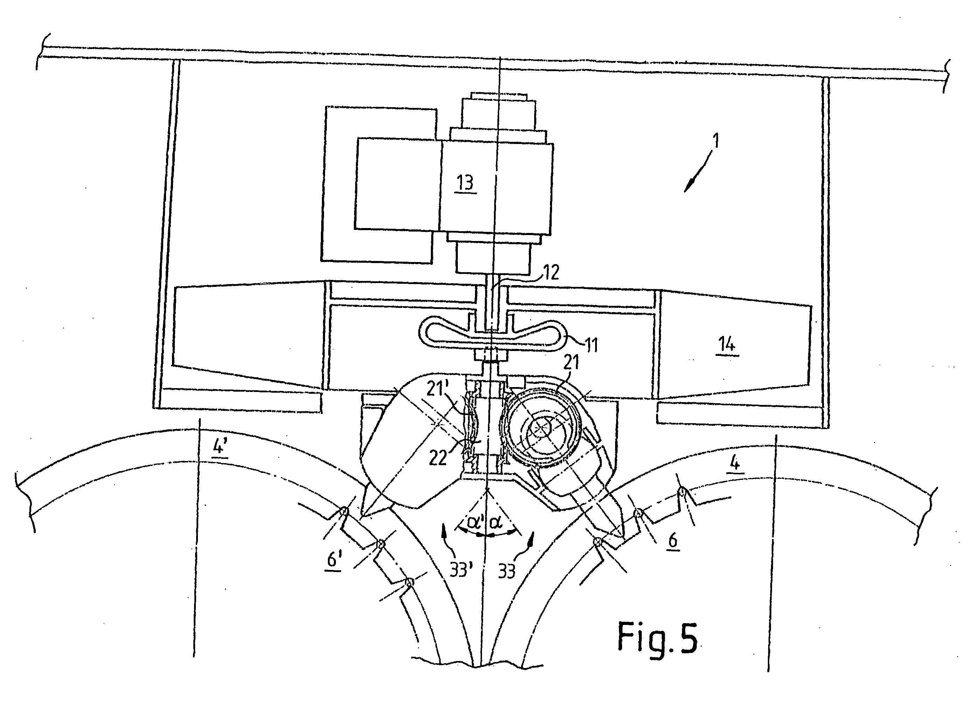

Der dargestellte Antrieb für den Plattenstapel 4 läßt sich auch außerhalb eines erfindungsgemäßen Duftverdunsters einsetzen. Das dargestellte System läßt sich ohne weiteres, auch in der nachfolgend beschriebenen Ausführung gem. Fig. 5, auf jeden rotierenden Gegenstand anwenden, bei dem ein erfindungsgemäßes Antriebssystem Vorteile bietet. Dies kann insbesondere bei chemischen Produktionsanlagen der Fall sein, wo ebenfalls häufig Walzen, Wellen oder dergleichen innerhalb eines Flüssigkeitsbades drehen müssen, und somit ebenfalls das Problem auftritt, daß der Antrieb nicht mit der ggf. aggressiven Flüssigkeit nicht oder nur punktuell in Berührung kommen darf.

In Fig. 5 ist eine weitere Ausführungsform eines erfindungsgemäßen Antriebs dargestellt. Hierbei stehen zwei Plattenstapel 4, 4' mit jeweils einem Zahnrad 6, 6' parallel zueinander. Sie werden über einen doppelten Hebelantrieb 33, 33' angetrieben. Die Hebelantriebe 33 entsprechen den bisherigen oben beschriebenen einfachen Hebelantrieben. Sie werden über eine gemeinsame Schnecke 22 und die beiden Schneckenräder 21, 21' angetrieben. Im vorliegenden Fall ist die Achse der Schnecke 22 und damit auch der Getriebeantriebswelle 10 senkrecht angeordnet, wobei die beiden Hebelantriebe 33, 33' unter einem Winkel α bzw. α' schräg zur senkrechten Achse der Getriebeantriebswelle stehen. Entsprechend ist auch das Flügelrad 14 sowie der Elektromotor mit senkrecht verlaufender Achse angeordnet.

Durch die Verwendung zweier paralleler Plattenstapel wird die benetzte Oberfläche insgesamt vergrößert, wobei nach wie vor nur ein Antriebsmotor und ein Ventilator notwendig ist. Durch die genannte räumliche Anordnung der Plattenstapel ist eine kompakte Bauweise bei hoher Effizienz eines Duftverdunsters möglich.
- 1: Duftverdunster
- 2: Gehäuse
- 3: Öffnungsklappe
- 4: Plattenstapel
- 5: Achse
- 6: Zahnrad
- 7: Hebel
- 8: Hebelspitze
- 9: Getriebegehäuse
- 10: Getriebeantriebswelle
- 11: elastische Kupplung
- 12: Motorantriebswelle
- 13: Elektromotor
- 14: Flügelrad
- 15: Duftstoffwanne
- 16: Schubladenhalterung
- 17: Drehachse
- 21: Schneckenrad
- 22: Schnecke
- 23: Exzenterantriebswelle
- 24: Exzenter
- 25: obere Gleitfläche
- 26: untere Gleitfläche
- 27: obere Führungsfläche
- 28: untere Führungsfläche
- 29: Steckhülse
- 30: Verdunstungsraum
- 31: Lüftungsschlitze
- 32: Blende
- 33: Hebelantrieb

## Patentansprüche

1. Antrieb für einen rotierenden Gegenstand, wie eine Walze, Welle, Plattenstapel oder dergleichen, insbesondere für einen Duftverdunster, mit wenigstens einem schwenkbar gelagerten, über eine Exzenterantriebswelle (23) angetriebenen Hebel (7, 7') der mit einem Zahnrad (6) in Eingriff steht, wobei Mittel (24 - 28) zur Lagerung und Führung des Hebels (7, 7') für eine in das Zahnrad (6) eingreifende und mitnehmende Vorwärtsbewegung des Hebels (7, 7') vorgesehen sind, dadurch gekennzeichnet, daß zur Durchführung einer Translations- und Kippbewegung des Hebels (7, 7') dieser an einer oberen und unteren Führungsfläche (27, 28) geführt ist.

2. Antrieb nach Anspruch 1, dadurch gekennzeichnet, daß der Hebel (7) mit seiner zum Zahnrad (6) hinweisenden Spitze (8) in das Zahnrad (6) eingreift und diese Spitze (8) des Hebels (7) in jeder Stellung des Hebels (7) nach unten ausgerichtet ist.

3. Antrieb nach einem der Ansprüche 1 oder 2, dadurch gekennzeichnet, daß die Vorrichtung zur Lagerung und Führung (24 bis 28) des Hebels für eine zwischen zwei Vorwärtsbewegungen des Hebels stattfindende Rückwärtsbewegung eingerichtet ist, bei der sich der Hebel (7) nicht mehr in Eingriff mit dem Zahnrad (6) befindet und dieses während der Rückwärtsbewegung nicht berührt.

4. Antrieb nach einem der Ansprüche 1 bis 3, dadurch gekennzeichnet, daß die Vorrichtung zur Lagerung und Führung des Hebels (7) Gleitflächen (25, 26) am Hebel (7) und eine Halterung (9) mit den oberen und unteren Führungsflächen (27, 28) umfaßt.

5. Antrieb nach einem der Ansprüche 1 bis 4, dadurch gekennzeichnet, daß der Hebel an einem Ende mit der Exzenterantriebswelle (23) über einen Exzenter (24) gelenkig verbunden ist.

6. Antrieb nach einem der Ansprüche 1 bis 5, dadurch gekennzeichnet, daß zwei Hebel (7, 7') vorhanden sind.

7. Antrieb nach einem der Ansprüche 1 bis 6, dadurch gekennzeichnet, daß die Exzenterantriebswelle (23) für den Hebel (7) mit einem Schneckenrad (21) versehen ist, in das eine Schnecke (22) eingreift.

8. Antrieb nach Anspruch 7, dadurch gekennzeichnet, daß die Schnecke (22) über eine elastische Kupplung (11) mit der Antriebswelle (12) eines Motors verbunden ist.

## Claims

1. Drive for a rotating article, such as a roller, shaft, plate stack or the like, in particular for a fragrance vaporiser, with at least one pivotably mounted lever (7, 7') driven by an eccentric drive shaft (23) and which meshes with a toothed wheel (6), means (24 to 28) for bearing and guiding the lever (7, 7') being provided for a forward movement of the lever (7, 7') which engages with and entrains the toothed wheel (6), characterised in that in order to carry out a translational and tilting movement of the lever (7, 7'), this is guided on an upper and lower guide face (27, 28).

2. Drive according to claim 1, characterised in that the lever (7) engages with the toothed wheel (6) with its tip (8) pointing toward the toothed wheel (6) and this tip (8) of the lever (7) is aligned downwards in each position of the lever (7).

3. Drive according to one of claims 1 or 2, characterised in that the device for bearing and guiding (24 to 28) the lever is equipped for a backward movement which takes place between two forward movements, in which the lever (7) no longer meshes with the toothed wheel (6) and does not contact this during the backward movement.

4. Drive according to one of claims 1 to 3, characterised in that the device for bearing and guiding the lever (7) comprises slide faces (25, 26) on the lever (7) and a holding device (9) with the upper and lower guide faces (27, 28).

5. Drive according to one of claims 1 to 4, characterised in that the lever is articulatedly connected at one end to the eccentric drive shaft (23) by an eccentric (24).

6. Drive according to one of claims 1 to 5, characterised in that there are two levers (7, 7').

7. Drive according to one of claims 1 to 6, characterised in that the eccentric drive shaft (23) for the lever (7) is provided with a worm wheel (21) in which a worm (22) engages.

8. Drive according to claim 7, characterised in that the worm (22) is connected to the drive shaft (12) of a motor by a resilient coupling (11).

## Revendications

1. Entraînement pour un objet rotatif, comme un rouleau, un arbre, une pile de disques ou analogue, en particulier pour un évaporateur de parfum, comportant au moins un levier (7, 7'), monté pivotant, entraîné par l'intermédiaire d'un arbre d'entraînement excentrique (23), levier qui est en engagement avec une roue dentée (6), des moyens (24 - 28) étant prévus pour monter et guider le levier (7, 7') pour un mouvement d'avance, d'entraînement et d'engagement dans la roue dentée (6), du levier (7, 7'),
caractérisé en ce que, pour effectuer un mouvement de translation et de basculement du levier (7, 7'), celui-ci est guidé sur une surface de guidage supérieure (27) et une surface de guidage inférieure (28).

2. Entraînement selon la revendication 1,
caractérisé en ce que le levier (7) s'engage, par sa pointe (8) en regard de la roue dentée (6), dans la roue dentée (6) et cette pointe (8) du levier (7), dans chaque position du levier (7), est orientée vers le bas.

3. Entraînement selon une des revendications 1 ou 2,
caractérisé en ce que le dispositif pour monter et guider (24 à 28) le levier est prévu pour un mouvement de recul ayant lieu entre deux mouvements d'avance du levier, pour lequel le levier (7) ne se trouve plus en engagement avec la roue dentée (6) et n'est pas en contact avec celle-ci pendant le mouvement de recul.

4. Entraînement selon une des revendications 1 à 3,
caractérisé en ce que le dispositif pour monter et guider le levier (7) comporte des surfaces de glissement (25, 26) sur le levier (7) et un support (9) ayant les surfaces de guidage supérieure et inférieure (27, 28).

5. Entraînement selon une des revendications 1 à 4,
caractérisé en ce que le levier est relié, de façon articulée, à une extrémité, à l'arbre d'entraînement excentrique (23) par l'intermédiaire d'un excentrique (24).

6. Entraînement selon une des revendications 1 à 5,
caractérisé en ce que deux leviers (7, 7') sont prévus.

7. Entraînement selon une des revendications 1 à 6,
caractérisé en ce que l'arbre d'entraînement excentrique (23) pour le levier (7) est muni d'une roue à denture hélicoïdale (21), dans laquelle s'engage une vis sans fin (22).

8. Entraînement selon la revendication 7,
caractérisé en ce que la vis sans fin (22) est reliée, par l'intermédiaire d'un accouplement élastique (11), à l'arbre d'entraînement (12) d'un moteur.
